# EUROPEAN PATENT APPLICATION

(11) **EP 2 792 295 A1**
(43) Date of publication of application: **22.10.2014**
(21) Application number: 12858271.5
(22) Date of filing: 17.12.2012
(51) Int. Cl.: A61B 3/10, A61B 3/12

(54) **LASER SCANNING METHOD FOR MEASURING IN VIVO AMOUNT OF SPECIFIC SUBSTANCE**

(30) Priority: 16.12.2011 JP 2011276295
(71) Applicant: Riken, Saitama 351-0198 (JP)
(72) Inventor: WADA, Satoshi, Wako-shi Saitama 351-0198 (JP); TSUBOTA, Kazuo, Tokyo 160-8582 (JP); SHINJO, Atsushi, Fujisawa-shi Kanagawa 252-8520 (JP)
(74) Representative: Körfer, Thomas
(86) International application number: PCT/JP2012/008035
(87) International publication number: WO 2013/088746

(57) **Abstract**

In a laser scanning method for measuring in vivo substances, wavelength of the anti-Stokes line is made greater than wavelength absorption band of the specific substance, the wavelength of the probe light is shifted from the wavelength of the anti-Stokes line by a shift amount of Raman scattering of the specific substance, and the wavelength of the Stokes light is shifted from the wavelength of the probe light by the shift amount of the Raman scattering of the specific substance. According to this, density (concentration and distribution) of lutein in an eye fundus is measured quantitatively and non-invasively from a signal strength level of the anti-Stokes line.1

## Description

### [TECHNICAL FIELD]

The present invention relates to a laser scanning method for measuring in vivo substances capable of quantitatively and non-invasively measuring concentration and distribution of lutein especially in an eye fundus.

### [BACKGROUND TECHNIQUE]

Age-related macular degeneration which is the highest cause of blindness and which increases with advancing age after fifty is a large social problem in the current long-lived society. It is considered that one of foundations of the start of symptoms is chronic inflammation, and if time and cost required for prevention thereof are taken into account, it is necessary to diagnose a high-risk group by health check. For eye fundus macular pigment which is a barometer thereof, there is no approved measuring method at the present time, and it is impossible to diagnose before the start of symptoms. If it is possible to judge the high-risk group by general health check and to judge effects of preventive care, it is possible to prevent blindness by the age-related macular degeneration and restrictions on the movements by the blindness, and it is possible to contribute to formation of healthy long-lived society. However, by the current inspection devices, it is not possible to make the above-described judgment.

Of entire age-related macular degeneration, the current medical treatment is effective only for exudative type age-related macular degeneration, and the effect of the medical treatment is merely to prevent the macular degeneration from worsening. As current methods for the diagnosis by which it is desired to realize early recognition, early medical treatment as well as prevention of start of symptoms, there are eye fundus inspection, eye fundus camera, optical coherence tomography (OCT), and angiography. However, although these methods for the diagnosis can be utilized for already exhibited symptoms cases, it is not possible to judge the high-risk group.

Recently, public awareness of the age-related macular degeneration is extremely increased by awareness campaign which is carried out through mass media and the like. Since a population of aged energetic people is large, and it is said that 80% of external information comes through eyes, conscious of securement of visual performance of aged person is high. There are many patients who come to hospital for very early diagnostics, but by the current inspection devices, it is not possible to diagnose unless start of symptoms is exhibited, and a doctor cannot diagnose the patient. By the current inspection devices for checking altered morphology of eye fundus, this problem cannot be solved.

By previous studies, relation between an amount of intake of lutein which is the macular pigment and start of symptoms of age-related macular degeneration was reported, and age-related eye disease study 2 (AREDS2) which is large scale clinical test carried out on the initiative of U.S. NIH for verification of the relation is ongoing. Measurement of eye fundus macular pigment is absolutely necessary for detecting the high-risk group and for judging effects of preventive care of medical treatment. However, this measuring method has not yet been established. It seems to be possible to measure in vivo lutein concentration by blood drawing, but there is a problem that this measurement is invasive, and it cannot be said that this measurement absolutely reflects an amount of macular pigment.

To check the amount of in vivo macular pigment, non-invasive pigment measuring technique is absolutely necessary, but there is no inevasible method at present. It is desired to develop this technique, and to combine this technique with the eye fundus camera and the optical coherence tomography (OCT) for seeing in vivo configuration which has been developing as ophthalmology heretofore, because this means that biotransformation which remained unknown by the current inspection method can be grasped.

Measuring devices of eye fundus which have been realized heretofore irradiate light through eye balls, and the eye fundus is measured utilizing its scattering light. As measuring principle of the conventional techniques, resonance Raman scattering or a measuring method based on simple absorption is utilized. According to the method of the resonance Raman scattering, signal strength is attenuated more than six digits as compared with strength of utilized probe laser. If damage on the eye fundus is taken into consideration, strong laser cannot be utilized. As a result, large signal strength cannot be obtained, and sufficient signal with variation in concentration of lutein cannot be obtained. Measurement concerning absorption and fluorescence, spreading degrees of respective spectrums are large, distinction with respect to other biological substance is not sufficient, and precise observation cannot be carried out. Therefore, sufficiently quantitative observation was not carried out. According to a method for irradiating the entire eye fundus or a relatively large spot with probe light at a time, sufficient space resolution could not be obtained. Such insufficient quantitative performance and space resolution performance are problems for early diagnostics of age-related macular degeneration.

For example, patent document 1 shows an eye fundus inspecting device for irradiating an eye fundus with laser and inspecting blood vessel on the eye fundus utilizing scattering light.

Patent document 2 proposes an in vivo substance amount measuring method for measuring an amount of glycated hemoglobin utilizing coherent anti-Stokes Raman scattering (CARS).

As described in patent document 2, CARS is one type of Raman scattering. The CARS is a phenomenon in which when two kinds of light having different frequencies, i.e., pump light (probe light (frequency ωp)) and Stokes light (frequency ωs) enter substance, scattering light of frequency of 2ωp-ωs is discharged. When ωp-ωs coincides with character frequency ωV of molecule, vibration modes of a large number of molecules are excited resonantly, and it is possible to obtain extremely strong and coherent scattering light with excellent directionality.

The Raman scattering is a phenomenon in which when substance is irradiated with incident light having frequency of ω1, weak scattering light having frequency of ω1+ωR or ω1-ωR appears. Here, ωR is character frequency by vibration mode of molecule. In Raman scattering spectrum, many spectrum lines derived from each of the vibration modes of molecules appears. Therefore, if spectrum is analyzed, it is possible to detect molecule.

### [PRIOR ART DOCUMENTS]

### [PATENT DOCUMENTS]

[Patent Document 1] Japanese Patent Application Laid-open No.2001-275976
[Patent Document 2] Japanese Patent Application Laid-open No.2010-5062

### [SUMMARY OF THE INVENTION]

### [PROBLEM TO BE SOLVED BY THE INVENTION]

Here, to explain quantitative performance, if electric field of probe laser is defined as Ep and quantum noise is defined as Q, dipole p=χQEpEp which is proportional to product QEp of quantum noise and electric field of probe laser is induced in Raman scattering which is conventional technique. Therefore, dipole electrically vibrates in plus and minus, thereby generating Stokes light.

A value of Q is 10⁻¹²W level. If damage on a human body is considered, laser which can be utilized for probe is 1mW (10⁻⁶W) or lower. It is said that a signal by this combination is attenuated about more than six digits as compared with pumped laser.

Until this point, Raman scattering light having a shift amount which is peculiar to lutein generated by irradiation of laser was measured. Therefore, to earn scattering strength, light around resonance line existing around 488nmwas utilized for excitation light. However, since scattering strength of Raman scattering was attenuated to about six digits, a strong signal could not be obtained.

It is an object of the present invention to provide a laser scanning method for measuring in vivo substances capable of measuring concentration and distribution of lutein in eye fundus quantitatively and non-invasively.

### [MEANS FOR SOLVING THE PROBLEM]

A first aspect of the present invention provides a laser scanning method for measuring in vivo substances in which specific substance in living organism is irradiated with probe light and Stokes light having different wavelengths, thereby generating anti-Stokes line, and density of the specific substance is measured from a signal strength level of the anti-Stokes line, wherein wavelength of the anti-Stokes line is made greater than wavelength absorption band of the specific substance, the wavelength of the probe light is shifted from the wavelength of the anti-Stokes line by a shift amount of Raman scattering of the specific substance, and the wavelength of the Stokes light is shifted from the wavelength of the probe light by the shift amount of the Raman scattering of the specific substance.

According to a second aspect of the invention, in the laser scanning method for measuring in vivo substances of the first aspect, the specific substance is lutein existing in an eye fundus, and the wavelength of the anti-Stokes line is 550 nm or more.

According to a third aspect of the invention, in the laser scanning method for measuring in vivo substances of the second aspect, the wavelength of the anti-Stokes line is 700 nm or less.

A fourth aspect of the invention provides a laser scanning device for measuring in vivo substances in which specific substance in living organism is irradiated with probe light and Stokes light having different wavelengths, thereby generating anti-Stokes line, and density of the specific substance is measured from a signal strength level of the anti-Stokes line, wherein the laser scanning device for measuring in vivo substances comprises a laser device using visible 2 wavelength laser by pulse operation, and a confocal optical system which focuses the probe light at the living organism and which detects only a signal generated from a light focused point, wavelength of the anti-Stokes line is made greater than wavelength absorption band of the specific substance, the wavelength of the probe light is shifted from the wavelength of the anti-Stokes line by a shift amount of Raman scattering of the specific substance, and the wavelength of the Stokes light is shifted from the wavelength of the probe light by the shift amount of the Raman scattering of the specific substance and this wavelength of the Stokes light is used.

### [EFFECT OF THE INVENTION]

The CARS can take out information (angular frequency Ω of vibration mode) of only measured specific substance by a difference in wavelengths between two laser light (probe light (angular frequency ω1) and Stokes light (angular frequency ω2)). Therefore, since an obtained signal is proportional to density of the specific substance, the density of the specific substance can be identified.

According to the present invention, it is possible to obtain wavelength of anti-Stokes light shifted by an amount of energy which is peculiar to specific substance called Raman shift by wavelength of the probe light by utilizing CARS, and a strong signal is obtained by this anti-Stokes light. Since signal light obtained by the wavelength of the anti-Stokes light is proportional to density of specific substance, it is possible to know density of the specific substance from proportionality coefficient of the signal and density.

According to the present invention, it is possible to measure concentration and distribution of lutein in an eye fundus quantitatively and non-invasively. According to this, from here on, it is possible to diagnose, as early as possible, reduction in lutein concentration of an eye fundus before developing age-related macular degeneration which is the highest factor of blindness, and to prevent the age-related macular degeneration by resupplying necessary nutrition.

### [BRIEF DESCRIPTION OF THE DRAWINGS]

Fig. 1 is a configuration diagram showing a laser scanning method for measuring in vivo substances according to an embodiment of the present invention;
Fig. 2 is an energy diagram of a CARS process; and
Fig. 3 is a diagram showing absorption characteristics of lutein according to a measurement result.

### [EXPLANATION OF SYMBOLS]

- 1: laser device
- 2: confocal optical system
- 3: pinhole
- a: probe light
- b: Stokes light
- c: macular region
- d: anti-Stokes line
- e: crystalline lens

### [BEST MODE FOR CARRYING OUT THE INVENTION]

A first aspect of the present invention provides a laser scanning method for measuring in vivo substances , wherein wavelength of the anti-Stokes line is made greater than wavelength absorption band of the specific substance, the wavelength of the probe light is shifted from the wavelength of the anti-Stokes line by a shift amount of Raman scattering of the specific substance, and the wavelength of the Stokes light is shifted from the wavelength of the probe light by the shift amount of the Raman scattering of the specific substance. According to the configuration of this aspect, it is possible to obtain wavelength of anti-Stokes light shifted by an amount of energy which is peculiar to specific substance called Raman shift by wavelength of the probe light by utilizing CARS, and a strong signal is obtained by this anti-Stokes light. Since signal light obtained by the wavelength of the anti-Stokes light is proportional to density of specific substance, it is possible to know density of the specific substance from proportionality coefficient of the signal and density.

According to a second aspect of the invention, in the laser scanning method for measuring in vivo substances of the first aspect, the specific substance is lutein existing in an eye fundus, and the wavelength of the anti-Stokes line is 550 nm or more. According to this aspect, it is possible to measure density of lutein existing in an eye fundus without being influenced by lutein existing on a crystalline lens.

According to a third aspect of the invention, in the laser scanning method for measuring in vivo substances of the second aspect, the wavelength of the anti-Stokes line is 700 nm or less. According to this aspect, it is possible to measure lutein within a range of visualization.

A fourth aspect of the invention a laser device using visible 2 wavelength laser by pulse operation, and a confocal optical system which focuses the probe light at the living organism and which detects only a signal generated from a light focused point, wavelength of the anti-Stokes line is made greater than wavelength absorption band of the specific substance, the wavelength of the probe light is shifted from the wavelength of the anti-Stokes line by a shift amount of Raman scattering of the specific substance, and the wavelength of the Stokes light is shifted from the wavelength of the probe light by the shift amount of the Raman scattering of the specific substance and this wavelength of the Stokes light is used. According to this aspect, it is possible to provide a device through which a user can know density of specific substance.

### [EMBODIMENT]

A laser scanning method for measuring in vivo substances according to an embodiment of the present invention will be described.

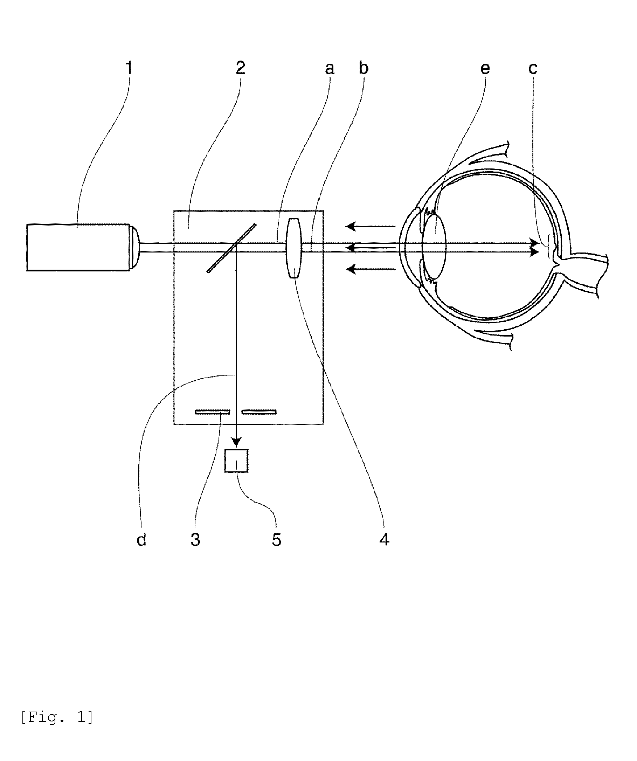

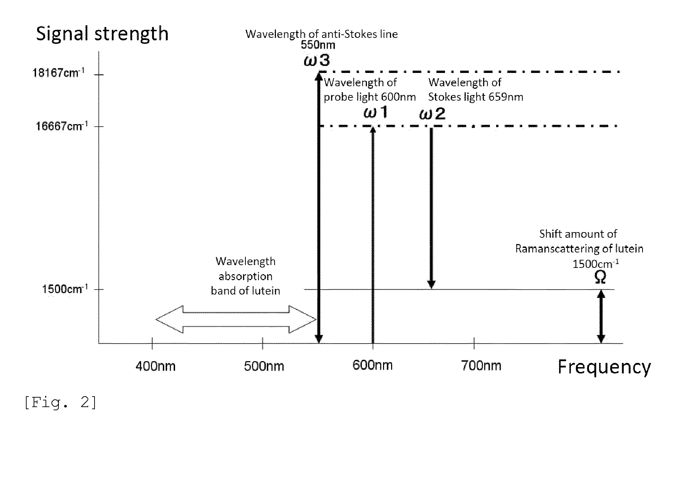

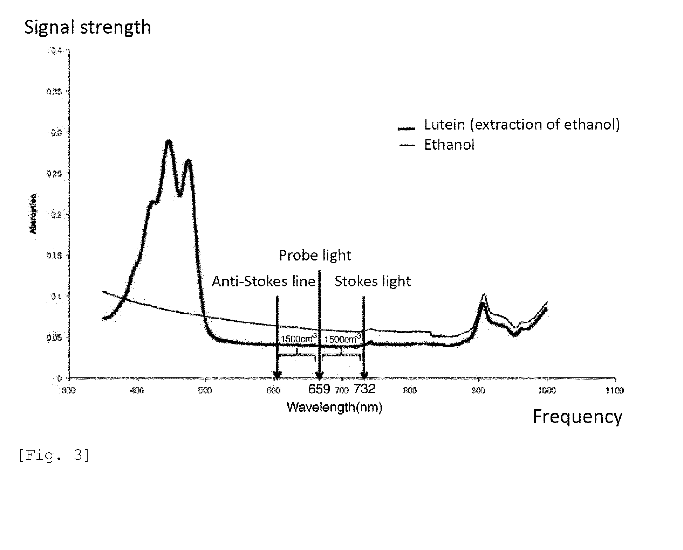

Fig. 1 is a configuration diagram showing the laser scanning method for measuring in vivo substances based on CARS spectroscopic method, Fig. 2 is an energy diagram of a CARS process, and Fig. 3 is a diagram showing absorption characteristics of lutein according to a measurement result.

As a laser device 1, visible 2 wavelength laser by pulse operation is used. In-plane resolution is set to 100 x 100µm, depth resolution is set to 20µm, and observation precision is set to optical concentration variation 3%. Galvanic laser scanning is used. Specific substance to be measured is lutein existing in an eye fundus. Titanium-sapphire laser is suitable for the laser device 1 for example.

If substance is irradiated with probe light (Ep electric field strength) a and Stokes light (Es electric field strength) b simultaneously, polarization of a product which is third non-linear polarization EsEpEp is created. This polarization vibrates with short wavelength by Raman shift amount which is peculiar to substance, and anti-Stokes line is forcibly generated by strong electric field formed by laser.

A macular region c of an eye fundus has macular pigment composed of lutein and zeaxanthin. This pigment exists for guarding macula which is most exposed to light. This pigment absorbs blue light which has highest energy, and has anti-oxidized stress ability. Therefore, this pigment prevents macula tissue damage. However, it is reported that the pigment is reduced with age, and this is related to start of symptoms of the age-related macular degeneration. It is reported that administration of lutein which is the macular pigment is conducive to prevention of the age-related macular degeneration. In this embodiment, macular pigment is measured in living organism.

A confocal optical system 2 focuses probe light a at living organism (eye fundus) and detects only a signal generated from a light focused point. On the opposite side from an objective lens 4, a virtual focus position is located and a pinhole 3 is placed. A detector 5 detects signal light (anti-Stokes line) d which passes through the pinhole 3, and this makes it possible to detect only light which is generated from the focus position. If the focus position is moved in a depth direction of the macular region c of the eye fundus, only a signal obtained from the focus position is detected as long as light reaches in the depth direction. Therefore, it is possible to obtain resolution performance in the depth direction of the macular region c. As a result, resolution performance in an in-plane and a depth direction of the macular region c is obtained. By scanning this irradiation position of the laser in an observation-desired region, information of all of the observation regions is obtained.

Utilizing laser scanning type confocal microscope system which is utilized in biology, a relation between wavelengths of two laser, signal strength obtained from lutein and lutein concentration is previously measured. At the same time, approximate density of lutein existing in an eye fundus is measured, and a relation between density of the lutein and a signal strength level is previously determined. The signal strength level and the density are proportional to each other.

The lutein which is to be detected in the embodiment is pigment having wavelength absorption band from wavelength of 400 nm to 550 nm. According to the measurement utilizing CARS, absolute strength of a signal is prevented from being changed by absorption of this pigment. To obtain information in the depth direction in an eye fundus, wavelength of pumped later (probe light) is set to in the near-infrared (including visible region close to the near-infrared), wavelength of anti-Stokes line is set to 550 nm or more, it is possible to avoid direct wavelength absorption band of lutein itself.

Lutein is included not only in retina, but also in crystalline lens e itself, and is also included in a path itself used for measuring light. Therefore, if wavelength absorption band is only seen directly, this point becomes a problem and thus, wavelength of the anti-Stokes line is set to 550 nm or more. A shift amount of Raman scattering of lutein is 1500 cm⁻¹. If wavelength of the anti-Stokes line is set to 550 nm, pumped laser corresponding to wavelength of probe light a of CARS utilizes wavelength of 600 nm which is shifted toward infrared side from wavelength of anti-Stokes line by 1500 cm⁻¹. The wavelength is shifted by optical parametric oscillation (OPO) or wavelength conversion method.

Further, as wavelength of the Stokes light b, wavelength of 659 nm which is shifted toward infrared side from wavelength of the probe light a by 1500 cm⁻¹ is utilized.

Fig. 3 shows that lutein has wavelength absorption band of wavelength from 400 nm to 500 nm. Wavelength of the probe light a is set to 659 nm and wavelength of the Stokes light b is set to 732 nm. As a result, wavelength of the anti-Stokes line can be set to 600 nm, density of lutein existing in an eye fundus can be measured without receiving influence of lutein existing in a crystalline lens, and it is possible to measure lutein within a range of visualization.

Wavelength of the anti-Stokes line is 550 nm in this embodiment, to avoid absorption by lutein, it is preferable that the wavelength of the anti-Stokes line is greater than 550 nm, e.g., 600 nm or more. In terms of visualization, it is preferable that the probe light a and the Stokes light b are set such that wavelength of the anti-Stokes line is 700 nm or lower. For example, if the wavelength of the probe light a is set to 730 nm and the wavelength of the Stokes light b is set to 816.6 nm, wavelength of the anti-Stokes line can be set to 660 nm.

Even if laser output which is necessary as the probe light a and the Stokes light b is set weaker than normal Raman scattering, obtained signal strength becomes higher than normal Raman scattering by several digits. Hence, the obtained output has sufficient strength. That is, if necessary dipole is replaced in optical four-wave mixing process in induction Raman scattering process and two laser is used, a shape of EpEpEs is obtained. Although this replacement is conducted between simple Q and Es (1 mW level), it is possible to create dipole which is stronger more than six digits as comparison with strength. That is, in theory, signal output which is stronger by six digits is obtained.

As described above, according to the present invention, pumped laser and laser light corresponding to Stokes line are emitted simultaneously with two wavelengths, third Raman polarization is forcibly produced, and anti-Stokes line of strong scattering strength is obtained. Approximately, it is possible to obtain a signal which is stronger than resonance scattering more than four digits. Further, laser is pulsed to earn electric field strength, and average strength of laser is reduced, thereby increasing signal strength. If magnitude of polarization is taken into account, a signal which is stronger than that obtained by a method of resonance scattering by about five digits can finally be obtained. Since this signal is proportional to lutein concentration, it is possible to known lutein concentration from the strength. Since the signal strength is sufficiently strong, it is possible to precisely measure with large dynamic range.

Although lutein is used as specific substance in this embodiment, the specific substance may be amino acid, nucleic acid, sugar group, fat or other substance existing in living organism.

### [INDUSTRIAL APPLICABILITY]

The present invention can be utilized for health check of age-related macular degeneration.

## Claims

1. A laser scanning method for measuring in vivo substances in which specific substance in living organism is irradiated with probe light and Stokes light having different wavelengths, thereby generating anti-Stokes line, and density of the specific substance is measured from a signal strength level of the anti-Stokes line, wherein
wavelength of the anti-Stokes line is made greater than wavelength absorption band of the specific substance,
the wavelength of the probe light is shifted from the wavelength of the anti-Stokes line by a shift amount of Raman scattering of the specific substance, and
the wavelength of the Stokes light is shifted from the wavelength of the probe light by the shift amount of the Raman scattering of the specific substance.

2. The laser scanning method for measuring in vivo substances according to claim 1, wherein the specific substance is lutein existing in an eye fundus, and the wavelength of the anti-Stokes line is 550 nm or more.

3. The laser scanning method for measuring in vivo substances according to claim 2, wherein the wavelength of the anti-Stokes line is 700 nm or less.

4. A laser scanning device for measuring in vivo substances in which specific substance in living organism is irradiated with probe light and Stokes light having different wavelengths, thereby generating anti-Stokes line, and density of the specific substance is measured from a signal strength level of the anti-Stokes line, wherein
the laser scanning device for measuring in vivo substances comprises a laser device using visible 2 wavelength laser by pulse operation, and a confocal optical system which focuses the probe light at the living organism and which detects only a signal generated from a light focused point,
wavelength of the anti-Stokes line is made greater than wavelength absorption band of the specific substance,
the wavelength of the probe light is shifted from the wavelength of the anti-Stokes line by a shift amount of Raman scattering of the specific substance, and
the wavelength of the Stokes light is shifted from the wavelength of the probe light by the shift amount of the Raman scattering of the specific substance and this wavelength of the Stokes light is used.
